# EUROPÄISCHE PATENTANMELDUNG

(11) **EP 1 674 056 A1**
(43) Veröffentlichungstag der Anmeldung: **28.06.2006**
(21) Anmeldenummer: 05405342.6
(22) Anmeldetag: 06.05.2005
(51) Int. Cl.: A61F 9/007

(54) **Set für chirurgisches Instrument für die Augenchirurgie und chirurgisches Instrument**

(30) Priorität: 27.12.2004 CH 21532004
(71) Anmelder: Medicel AG, 9443 Widnau (CH)
(72) Erfinder: Hohl, Emil, 9435 Heerbrugg (CH)
(74) Vertreter: Riederer, Conrad A.

(57) **Zusammenfassung**

Das Set zur Verwendung mit einem Handstück (41) weist in einer sterilen Verpackung zwei Baugruppen (11,13) auf. Die erste Baugruppe umfasst die Hohlnadel (15), das Röhrchen (17) und den Verschluss (19). Die zweite Baugruppe besteht aus dem Anschlussteil (25) und der darin angeordneten Membran (33), die eine Durchgangsöffnung (35) für das Röhrchen (17) aufweist. Nach dem Einsetzen der beiden Baugruppen in das Handstück (41) ragt das Röhrchen (17) durch die Durchgangsöffnung (35). Die Membran (33) erlaubt eine Vibrationsbewegung des Röhrchens (17) während der Operation und bildet zudem eine Dichtung, die das Eindringen von Flüssigkeit in das Innere des Handstücks (41) verhindert. Nach der Operation wird beim Entfernen des Anschlussteils (25) am Röhrchen anhaftende Flüssigkeit abgestreift und nach dem Einsetzen des Verschlusses (19) kann keine Flüssigkeit aus dem Röhrchen ausfliessen, wenn die erste Baugruppe aus dem Handstück herausgezogen und entsorgt wird. Da im System fliessende Aspirationsflüssigkeit während der Operation nie in Kontakt mit dem Handstück 41 gelangt, besteht eine hohe Sicherheit für den Patienten, die sonst nur duch vollständige Anwendung von Einweg-Produkten erreicht werden kann.

## Beschreibung

Die Erfindung betrifft ein Set zur Verwendung mit einem Handstück eines chirurgischen Instruments für die Augenchirurgie.

Die WO-A-00/53136 beschreibt ein chirurgisches Instrument für die Augenchirurgie. Dieses Instrument besitzt ein Handstück, das im Innern einen zentralen Kanal aufweist. Vorn am Handstück ist eine Hohlnadel angeordnet, welche mit diesem Kanal in Verbindung steht. Am hinteren Ende des Handstücks mündet der Kanal in ein Anschlussteil zum Anschluss an einen Aspirationsschlauch. Die Hohlnadel selbst ist von einer Manschette, dem sogenannten Irrigationsaufsatz, umgeben, welcher der Zufuhr von Spülflüssigkeit in den Bereich der Nadelspitze dient. Das Handstück weist auch einen Anschluss für den Spülflüssigkeitsschlauch auf. Piezokkristallscheiben sind mit der Hohlnadel gekoppelt, um diese in Längsrichtung zu vibrieren. Das Instrument dient zur Entfernung der Augenlinse vor dem Einsetzen einer intraokularen Linse. Das Zerstören der Augenlinse erfolgt durch die Vibration der Hohlnadel. Die durch das Instrument zugeführte Spülflüssigkeit wird zusammen mit den herausgelösten Gewebeteilen durch das Handstück hindurch abgesaugt. Es kann nun vorkommen, dass durch Gewebeteile im Handstück oder in der daran angeschlossenen Aspirationsleitung eine Verstopfung entsteht und die Absaugung nicht mehr funktioniert. Um die Verstopfung zu lösen, kann der Operateur einen kurzen Rückfluss auslösen. Dadurch kann die Aspirationsflüssigkeit aus dem Handstück ins Auge gelangen. Dies kann zu einer Infektion führen, wenn das Handstück im Innern nicht absolut steril war. Es kann nämlich nicht ausgeschlossen werden, dass sich im Innern des Handstücks von einer früheren Operation her Verunreinigungen befinden, die durch das Reinigungs- und Sterilisationsverfahren nicht vollständig beseitigt wurden. Dies trifft insbesondere für Prionen zu. Prionen sind z.Bsp. die Ursache für die Kreutzfeld-Jakobsche Krankheit.

In der US-4 386 927 wird vorgeschlagen, die Hohlnadel an einen Schlauch anzuschliessen der durch das Handstück verläuft. Dieses Handstück sieht aber keine Mittel vor, um die Hohlnadel zu vibrieren.

Bereits im Jahre 1999 brachte die Firma Oertli Instrumente AG, CH-9442 Berneck, ein chirurgisches Instrument für Mikrochirurgie und Pars Plana Vitrektomie auf den Markt, das ein Handstück mit einem Magnetmotor aufweist, der bis zu 1200 Schwingungen pro Minute ausführt. In das Handstück kann ein Einwegschneidkopf eingesetzt werden, der eine Hohlnadel aufweist, welche eine Verlängerung in Form eines Röhrchens aufweist, das sich durch das Handstück hindurch erstreckt. An das Ende des Röhrchens kann der Aspirationsschlauch angeschlossen werden. Trotzdem kann eine Kontamination des Handstücks nicht vermieden werden, weil bei der Entnahme der Hohlnadel Aspirationsflüssigkeit aus dem Röhrchen ausfliessen und den Kanal im Handstück verunreinigen kann. Eine Publikation bezüglich des beschriebenen Instruments findet sich in "Augenblicke I / 2002", publiziert von Mediconsult AG, CH-9325 Roggwil.

Auch das chirurgische Instrument der WO03/073969 ist ähnlich wie das Instrument der Firma Oertli Instrumente AG aufgebaut. Es ist ein Schlauchstück an der Hohlnadel angeschlossen und erstreckt sich durch das Handstück hindurch zum Anschluss für den Aspirationsschlauch. Im Gegensatz zu den Aussagen in der zitierten Druckschrift kann aber nicht vermieden werden, dass das Innnere des Handstücks nicht mit Aspirationsflüssigkeit und abgesaugten Gewebebestandteilen in Kontakt gelangt. Damit die Sonotrode die Hohlnadel richtig vibrieren kann, sollte sich der Schlauch in Längsrichtung bewegen können. Dies ist bei der gezeigten Konstruktion des Handstücks nur möglich, wenn im hinteren Abschlussteil des Handstücks die Durchgangsöffnung für den Schlauch gross bemessen ist, dass sich der Schlauch darin praktisch ohne Widerstand in Längsrichtung bewegen kann. Bei einer so bemessenen Durchgangsöffnung kann aber Aspirationsflüssigkeit in das Innere des Handstücks eindringen und dieses dort kontaminieren. Ferner kann der Kanal im Handstück auch kontaminiert werden, wenn die Hohlnadel zusammen mit dem daran befestigten Schlauchstück aus dem Handstück herausgezogen wird. Bei dieser Gelegenheit kann nämlich aus dem Ende des im Schlauchstücks verbliebene Aspirations-Flüssigkeit zusammen mit darin enthaltenen Gewebebestandteilen heraustropfen. Diese Probleme dürften denn auch der Grund dafür sein, dass kein Instrument dieser Bauart im Handel ist.

Es ist daher Aufgabe der vorliegenden Erfindung ein chirurgisches Instrument zu schaffen, bei welchem keine Gefahr besteht, dass das Handstück durch Aspirationsflüssigkeit im schlecht zu reinigenden Innern kontaminiert wird bzw. mit Aspirationsflüssigkeit Verunreinigungen aus dem Innern des Handstücks zur Hohlnadel gelangen können. Insbesondere soll ein Set zur Verwendung mit einem Handstück eines chirurgischen Instruments geschafffen werden, das dies ermöglicht.

Zur Lösung dieser Aufgabe dient ein Set, das gekennzeichnet ist durch eine erste Baugruppe, welche eine Hohlnadel, ein daran angeschlossenes Röhrchen und ein Röhrchenverschluss am freien Ende des Röhrchens umfasst und eine zweite Baugruppe, welche ein an das Handstück anschliessbares Anschlussteil mit einem darin befestigten Membran umfasst, welche eine Durchgangsöffnung für das Röhrchen aufweist.

Die Verwendung eines solchen Sets ermöglicht es dem Operateur, nach der Operation das Anschlussteil zu entfernen, wobei durch die Membran am hinteren Teil des Röhrchens anhaftende Flüssigkeit abgestreift wird. Das Röhrchenende kann dann mit dem Röhrchenverschluss verschlossen werden, so dass keine Aspirationsflüssigkeit mit Gewebeteilen ausfliessen kann, wenn die Hohlnadel losgeschraubt und samt Röhrchen und Verschluss aus dem Instrument herausgezogen und entsorgt wird.

Es sind verschiedene Ausbildungen des Röhrchenverschlusses möglich. Zweckmässigerweise ist der Röhrchenverschluss als Pfropfen ausgebildet. Dies ist kostengünstig und erlaubt eine einfache Handhabung. Der Pfropfen kann einen Griffteil aufweisen, der aus dem Röhrchen ragt und einen Durchmesser besitzt, der geringfügig grösser als der Durchmesser des Röhrchens ist. Der Griffteil ermöglicht eine leichte Handhabung, und wegen des grösseren Durchmessers des Griffteils kommt das Röhrchenende beim Herausziehen des Röhrchens nie in Berührung mit den Kanalwänden im Innern des Handstücks.

Der Griffteil kann gegen sein Ende hin konisch verjüngt sein. Dadurch wird einerseits das Aufsetzen des Anschlussteils mit der darin angeordneten Membran auf das Handstück erleichtert und andererseits kommt beim Herausziehen der Hohlnadel und dem daran angeschlossenen Röhrchen der hintere Teil des Pfropfens nicht in Berührung mit den Wänden des Kanals im Inneren des Handstücks.

Vorteilhaft weist die Membran eine Dichtlippe auf, wobei der Durchmesser der Durchgangsöffnung etwas kleiner als der Durchmesser des Röhrchens ist. Dadurch wird eine gute Dichtung gewährleistet und beim Entfernen des Anschlussteils wird durch die Dichtlippe am hinteren Teil des Röhrchens anhaftende Flüssigkeit abgestreift.

Die Membran hat vorteilhaft die Form einer dünnen Scheibe. Eine solche Ausbildung hat den Vorteil, dass die Vibrationsbewegung von Hohlnadel und Röhrchen durch das Membran nur geringfügig gedämpft wird.

Die Befestigung der Membran im Anschlussteil kann auf verschiedene Weise erfolgen, z.B. durch Einpressen, Verleimen oder Verschweissen oder mittels eines Befestigungsrings. Bei der Verwendung eines Befestigungsrings ist es auch möglich, die Membran schon vor der Montage im Anschlussteil mit dem Befestigungsring zu verbinden und dann diesen beispielsweise durch Einpressen im Anschlussteil zu befestigen.

Der Befestigungsring kann eine zur Membran konisch verlaufende Oeffnung aufweisen, um beim Befestigen des Anschlussteils am Handstück das Röhrchen zu zentrieren und sicher durch die Durchgangsöffnung der Membran hindurchzuführen.

Die Dichtlippe kann beispielsweise einen keilförmigen Querschnitt aufweisen. Dies ermöglicht eine gute Reinigungswirkung.

Als Material für die Membran eignet sich vorteilhaft Silikongummi.

Es sind verschiedene Formgebungen für das Anschlussteil möglich. Zweckmässigerweise besitzt jedoch das Anschlussteil die Form eines "Luer"-Adaptors, wie sie bei solchen chirurgischen Instrumenten üblich sind.

Die Erfindung betrifft auch die Verwendung des Sets mit einem Handstück. Dabei wird die erste Baugruppe von vorn in das Handstück eingeführt und mit diesem z.B. durch Einschrauben befestigt. Dann wird die zweite Baugruppe hinten auf das Handstück aufgesetzt und der Röhrchenverschluss entfernt. Das Instrument ist nun zum Anschluss an den Spülflüssigkeits- und den Aspirationsschlauch bereit.

Die Erfindung betrifft nicht nur ein Set, sondern auch ein chirurgisches Instrument. Dieses Instrument besitzt ein Handstück, das im Innern ein Kanal aufweist, ein Anschluss für den Spülflüssigkeitsschlauch, ein Anschluss für den Aspirationsschlauch, und eine am Handstück befestigbare Hohlnadel und ein an der Hohlnadel befestigtes Röhrchen, welches zum hinteren Ende des Handstücks führt. Erfindungsgemäss ist das chirurgische Instrument dadurch gekennzeichnet, dass am hinteren Ende des Handstücks ein wegnehmbares Anschlussteil für den Aspirationsschlauch angeordnet ist, in welchem sich eine Membran befindet, durch die sich das Röhrchen erstreckt.

Ein Ausführungsbeispiel der Erfindung wird unter bezugnahme auf die Zeichnung beschrieben. Es zeigt:
- Figur 1:: eine erste Baugruppe des Sets,
- Figur 2:: die Teile, aus denen sich die erste Baugruppe zusammensetzt,
- Figur 3a:: die zweite Baugruppe,
- Figur 3b:: im vergrösserten Massstab eine weitere Ausführungsform der zweiten Baugruppe,
- Figur 3c:: in vergrössertem Massstab eine dritte Ausführungsform der zweiten Baugruppe,
- Figur 4:: eine Ansicht von links der Baugruppe von Figur 3a, jedoch ohne eingesetzte Membran,
- Figur 5a:: in vergrössertem Massstab einen Schnitt durch die in Figur 3a ersichtliche Membran,
- Figur 5b:: in vergrössertem Massstab einen Schnitt durch die in Figur 3b ersichtliche Membran,
- Figur 6:: das chirurgische Instrument und
- Figur 7:: einen vergrösserten Abschnitt von Figur 6.

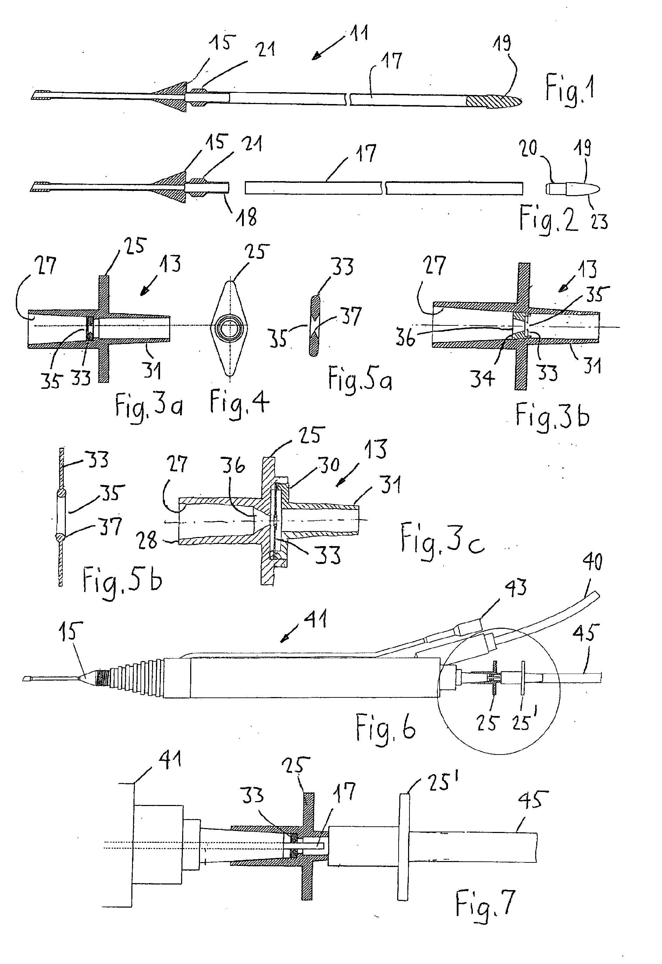

Wie die Figuren 1 bis 5 zeigen, besteht das Set aus einer ersten Baugruppe 11 und einer zweiten Baugruppe 13.

Die erste Baugruppe 11 umfasst eine Hohlnadel 15, ein vorzugsweise flexibles Röhrchen 17 und einen Röhrchenverschluss 19. Die Hohlnadel, die vorzugsweise aus Titan besteht, besitzt einen Ansatz 18 zur Befestigung des Röhrchens 17. Zur Befestigung der Hohlnadel 15 am Handstück kann in üblicher Weise ein Gewinde 21 dienen. Das Röhrchen 17 besteht aus flexiblem Kunststoff, z.Bsp. Polyamid. Der Röhrchenverschluss 19 ist als Pfropfen ausgebildet und weist einen Dichtungsteil 20, der satt in das Röhrchen 17 passt, und einen Griffteil 23 auf. Der Griffteil 23 ragt nach dem Einsetzen des Pfropfens 19 aus dem Röhrchen 17 heraus und besitzt einen Durchmesser, der geringfügig grösser ist als der Durchmesser des Röhrchens 17. Der Griffteil 23 ist gegen sein Ende hin konisch verjüngt. Der Pfropfen besteht aus Kunststoff, z.Bsp. ABS.

Die in Figur 3a dargestellte zweite Baugruppe 13 besteht aus dem Anschlussteil 25, der die Form eines handelsüblichen "Luer"-Adaptors aufweist und einer Membran 33. "Luer"-Adaptoren besitzen einen Innenkonus 27 zum Aufstecken auf den Konus 29 (Fig. 7) eines Handstücks 41 und einen Konus 31 zum Anschliessen eines Aspirationsschlauchs 45. Der Konus 31 hat die gleiche Konizität wie der Innenkonus 27, so dass auf den Konus 31 auch ein weiterer "Luer"-Adaptor 25' aufgesteckt werden kann (Fig. 7). Die Membran 33 ist im Innern des Anschlussteils 25 befestigt. Die Befestigung erfolgt beispielsweise durch Einpressen oder Festkleben. Die Membran 33 besitzt eine Durchgangsöffnung 35, die kleiner als der Durchmesser des Röhrchens ist. Die Dichtlippe 37 besitzt einen keilförmigen Querschnitt. Als Material für die Dichtung eignet sich insbesondere Silikongummi.

In Fig. 3b ist eine zweite Ausführungsform der zweiten Baugruppe 13 dargestellt. Hier besitzt die Membran 33 die Form einer dünnen runden Scheibe (Fig. 5b). Die Dichtlippe 37 weist einen runden Querschnitt auf. Sie kann aber auch z.Bsp. einen keilförmigen oder anderen geeigneten Querschnitt besitzen. Der Befestigung der Membran 33 dient ein Befestigungsring 34. Dieser besitzt eine konisch zur Membran 33 verlaufende Öffnung 36, um das Röhrchen 17 zu zentrieren und sicher durch die Durchgangsöffnung 35 der Membran 33 hindurchzuführen.

Eine weitere Ausführungsform der zweiten Baugruppe 13 zeigt die Fig. 3c. Der Adaptor 25 hat die gleiche Funktion wie jener von Fig. 3a, besteht aber aus zwei meinteinander verbundenen Teilen 28, 30, zwischen welchen die Membran 33 angeordnet ist. Diese Bauart hat den Vorteil, dass die Membran 33 einen grösserern Durchmesser aufweisen kann, was sich günstig für das Schwingungsverhalten der Membran 33 auswirkt.

Selbstverständlich kann der Schlauch 45 - anders als in Fig. 7 gezeigt - direkt am Anschlussteil 25 angeschlossen werden. Die in Fig. 7 gezeigte Anschlussart hat aber den Vorteil, dass sie den Anschluss von verschieden gearteten Schlauchsystemen ermöglicht.

Die beiden Baugruppen 11 und 13 befinden sich bis zu ihrer Verwendung in einer sterilen Verpackung, z.Bsp. einer Blisterverpackung.

Vor der Operation werden die beiden Baugruppen 11,13 der sterilen Verpackung entnommen. Zuerst wird die Baugruppe 11, also Hohlnadel 15 mit Röhrchen 17 und Verschluss 19, in das Phako-Handstück 41 eingeführt und verschraubt (Fig. 6). Hierauf wird die Baugruppe 13, also der Anschlussteil 25 mit der Membran 33, auf den Konus 25 des Phako-Handstücks 41 aufgesetzt. Der hintere Teil des Röhrchens 17 wird dabei zwangsläufig durch die Durchgangsöffnung 35 der Membran 33 geführt. Die Membran 33 hat die Aufgabe während der Operation eine Abdichtung des Handstücks 41 gegenüber der Aspirationsflüssigkeit zu bewirken. Die Membran 33 gestattet aber dennoch ein Vibrieren der Hohlnadel 15 samt dem Röhrchen 17. Nach dem Aufstecken des Anschlussteils 25 und der Entnahme des Verschlusses 19 ist das chirurgische Instrument vollständig (Fig. 6).

Nach dem Anstecken der elektrischen Leitung 40 an den Impulsgenerator, dem Anschliessen der Spülflüssigkeitsleitung 43 an die Spülflüssigkeitsquelle, dem Anstecken des "Luer"-Adapters 25' mit dem daran angeschlossenen Aspirationsschlauch 45 und dem Füllen des Systems, kann die Operation durchgeführt werden.

Bei der Operation fliesst in bekannter Weise Spülflüssigkeit durch die Spülflüssigkeitsleitung 43 zum Handstück 41, wo sie über einen Irrigationsaufsatz (nicht dargestellt) zur Spitze der Hohlnadel 15 geführt wird, um von dort zusammen mit herausgelösten Gewebebestandteilen durch die Hohlnadel 15, das Röhrchen 17 und den Aspirationsschlauch 45 abgesaugt zu werden.

Nach der Operation wird der Adapter 25 samt Adapter 25' und Aspirationsschlauch 45 vom Handstück 41 entfernt und der Verschluss 19 auf das Röhrchen aufgesetzt. Danach wird die Hohlnadel 15 abgeschraubt und samt Röhrchen 17 und Verschluss aus dem Instrument herausgezogen und entsorgt.

Das Handstück 41 kommt innen weder während noch nach der Operation mit aspirierter Flüssigkeit in Kontakt. Somit besteht - im Gegensatz zu gewissen chirurgischen Instrumenten gemäss dem bisherigen Stand der Technik - keine Gefahr, dass z.Bsp. Prionen bei einer Operation in das Handstück gelangen, trotz Sterilisation im Handstück verbleiben und bei einer folgenden Operation eine Gefahr für den Patienten darstellen.

Weil das Handstück 41 im Innern nicht mit Aspirationsflüssigkeit in Kontakt kommt, ist der Reinigungsaufwand gering und die Sterilisation kann massiv vereinfacht werden.

### Zusammenfassend kann folgendes festgehalten werden:

Das Set zur Verwendung mit einem Handstück 41 weist in einer sterilen Verpackung zwei Baugruppen 11,13 auf. Die erste Baugruppe umfasst die Hohlnadel 15, das Röhrchen 17 und den Verschluss 19. Die zweite Baugruppe besteht aus dem Anschlussteil 25 und der darin angeordneten Membran 33, die eine Durchgangsöffnung 35 für das Röhrchen 17 aufweist. Nach dem Einsetzen der beiden Baugruppen in das Handstück 41 ragt das Röhrchen 17 durch die Durchgangsöffnung 35. Die Membran 33 erlaubt eine Vibrationsbewegung des Röhrchens 17 während der Operation und bildet zudem eine Dichtung, die das Eindringen von Flüssigkeit in das Innere des Handstücks 41 verhindert. Nach der Operation wird beim Entfernen des Anschlussteils 25 am Röhrchen anhaftende Flüssigkeit abgestreift und nach dem Einsetzen des Verschlusses 19 kann keine Flüssigkeit aus dem Röhrchen ausfliessen, wenn die erste Baugruppe aus dem Handstück herausgezogen und entsorgt wird. Da im System fliessende Aspirationsflüssigkeit während der Operation nie in Kontakt mit dem Handstück 41 gelangt, besteht eine hohe Sicherheit für den Patienten, die sonst nur duch vollständige Anwendung von Einweg-Produkten erreicht werden kann.

## Patentansprüche

1. Set zur Verwendung mit einem Handstück (41) eines chirurgischen Instruments für die Augenchirurgie, **gekennzeichnet durch** eine erste Baugruppe (11), welche eine Hohlnadel (15), ein daran angeschlossenes Röhrchen (15) und einen Röhrchenverschluss (19) am freien Ende des Röhrchens (17) aufweist, und eine zweite Baugruppe (13), welche ein an das Handstück (41) anschliessbares Anschlussteil (25) mit einer darin angeordneten Membran (33) aufweist, die eine Durchgangsöffnung (35) für das Röhrchen (17) besitzt.

2. Set nach Anspruch 1, **dadurch gekennzeichnet, dass** der Röhrchenverschluss (19) als Propfen ausgebildet ist.

3. Set nach Anspruch 2, **dadurch gekennzeichnet, dass** der Pfropfen (19) einen Dichtungsteil (20) und einen Griffteil (23) aufweist, der aus dem Röhrchen (17) ragt und einen Durchmesser besitzt, der geringfügig grösser als der Durchmesser des Röhrchens (17) ist.

4. Set nach Anspruch 3, **dadurch gekennzeichnet, dass** der Griffteil (23) gegen sein Ende hin konisch verjüngt ist.

5. Set nach einem der Ansprüche 2 bis 4, **dadurch gekennzeichnet, dass** der Pfropfen (19) aus Kunststoff, vorzugsweise ABS, besteht.

6. Set nach einem der Ansprüche 1 bis 5, **dadurch gekennzeichnet, dass** das Röhrchen (17) aus Kunststoff, vorzugsweise aus Polyamid, besteht.

7. Set nach einem der Ansprüche 1 bis 6, **dadurch gekennzeichnet, dass** die Membran (33) eine Dichtlippe (37) aufweist, und dass der Durchmesser der Durchgangsöffnung (35) geringfügig kleiner als der Durchmesser des Röhrchens (17) ist.

8. Set nach einem der Ansprüche 1 bis 6, **dadurch gekennzeichnet, dass** die Membran (33) die Form einer dünnen Scheibe aufweist.

9. Set nach einem der Durchmesser 1 bis 8, **dadurch gekennzeichnet, dass** die Membran (33) mit einem Befestigungsring im Anschlussteil befestigt ist.

10. Set nach einem der Ansprüche 1 bis 8, **dadurch gekennzeichnet, dass** das Anschlussteil (25) aus zwei miteinander verbundenen Teilen (28), (30) besteht, zwischen welchen die Membran (33) angeordnet ist.

11. Set nach einem der Ansprüche 1 bis 20, **dadurch gekennzeichnet, dass** der Befestigungsring eine zur Membran hin konisch verlaufende Oeffnung aufweist.

12. Set nach einem der Ansprüche 8 bis 11, **dadurch gekennzeichnet, dass** die Dichtlippe (37) einen keilförmigen Querschnitt aufweist.

13. Set nach einem der Ansprüche 8 bis 11, **dadurch gekennzeichnet, dass** die Dichtlippe (37) einen praktisch runden Querschnitt aufweist.

14. Set nach einem der Ansprüche 8 bis 11, **dadurch gekennzeichnet, dass** die Membran (33) aus Silikongummi besteht.

15. Set nach einem der Ansprüche 1 bis 14, **dadurch gekennzeichnet, dass** das Anschlussteil (25) die Form eines "Luer"-Adaptors aufweist.

16. Set nach einem der Ansprüche 1 bis 15, **dadurch gekennzeichnet, dass** das Anschlussteil (25) aus Kunststoff, vorzugsweise aus ABS, besteht.

17. Set nach einem der Ansprüche 1 bis 16, **dadurch gekennzeichnet, dass** die beiden Baugruppen (11;13) steril verpackt sind.

18. Verwendung des Sets nach einem der Ansprüche 1 bis 17 mit einem Handstück (41), **dadurch gekennzeichnet, dass** die erste Baugruppe (11) vorn in das Handstück (41) eingeführt und mit diesen befestigt wird, dass dann die zweite Baugruppe (13) hinten auf das Handstück (41) aufgesetzt wird, und dass der Röhrchenverschluss (19) entfernt wird, worauf das Instrument zum Anschluss an den Spülflüssigkeits- und den Aspirationsflüssigkeitsschlauch (43,45) bereit ist.

19. Chirurgisches Instrument für die Augenchirurgie, mit
einem Handstück (41), das im Innern einen Kanal aufweist,
einen Anschluss für den Spülflüssigkeitsschlauch (43),
einen Anschluss für den Aspirationsflüssigkeitsschlauch (45), und
einer am Handstück einsetzbaren Hohlnadel (15) und einem an der Hohlnadel (15) befestigten Röhrchen (17), welches zum hinteren Ende des Handstücks (41) führt, **dadurch gekennzeichnet, dass** am hinteren Ende des Handstücks (41) ein wegnehmbares Anschlussteil (25) für den Aspirationsschlauch (45) angeordnet ist, in welchem sich eine Membran (33) mit einer Durchgangsöffnung (35) befindet, durch die sich das Röhrchen (17) erstreckt.

20. Chirurgisches Instrument nach Anspruch 19, hergestellt unter Verwendung eines Sets gemäss den Ansprüchen 1 bis 17.
